# EUROPEAN PATENT APPLICATION

(11) **EP 1 676 570 A1**
(43) Date of publication of application: **05.07.2006**
(21) Application number: 05462003.4
(22) Date of filing: 12.07.2005
(51) Int. Cl.: A61K 31/198, A61K 31/522, A61K 31/353, A61K 31/05, A61K 31/35, A61K 8/30, A61K 36/00, A61P 21/02, A61P 21/06, A61P 9/14

(54) **Topical pharmaceutical compositions comprising L-arginine and uses thereof**

(30) Priority: 15.07.2004 HU 0401422
(71) Applicant: Mészáros, Lászlo, 2151 Fot (HU)
(72) Inventor: Mészáros, G. László, 2151 Fót (HU); Fuentes, Jesus, Hesperia, CA 92345 (US)
(74) Representative: Asboth, Domokos

(57) **Abstract**

The invention relates to a topical pharmaceutical composition such as a cream or an ointment for the improvement of the conditions of venous insufficiencies or skeletal muscles comprising as the active agent L-arginine or any pharmacologically acceptable L-arginine derivate together with conventional excipients and/or caffeine and/or vasoactive herbal ingredients and uses thereof.

Animal experiments and a limited number of human trials indicate that L-arginine is rapidly absorbed through the skin and reaches the bloodstream within minutes, after which L-arginine (alone or in combination with other vasoactive compounds) affects blood vessels in cutaneous as well as in subcutaneous tissues. As disclosed, the benefits of applying L-arginine onto the skin (alone or in combination with other vasoactive agents) include: the improvements of (*1*) the conditions of venous insufficiencies (pains, aching, inflammation, swelling, edema), (*2*) the conditions of skeletal muscles (muscle fatigue, spasms, cramps, pains) and (*3*) (in the case of long-term application) the appearance of the aging skin (reduced depth of wrinkles and increased softness of the skin).

## Description

### 1. Field of the Invention

The present invention relates to topical pharmaceutical compositions such as a cream or an ointment for the improvement of the conditions of venous insufficiencies or skeletal muscles comprising as the active agent L-arginine or any pharmacologically acceptable L-arginine derivate together with conventional excipients and/or caffeine and/or vasoactive herbal ingredients.

The topical skin application of the compositions according to the invention is used in creams or other ointments to improve conditions of varicose veins and the skeletal muscle. The invention also relates to such application of L-arginine (or its derivatives) to improve the esthetic appearance of the skin, especially that of the aging skin. All these applications are based on L-arginine being able to rapidly penetrate the skin (when applied in sufficient concentrations), thereby improving local blood supply in the skin as well as in subcutaneous tissues.

### 2. Prior Art

It is known from the literature that nitric oxide (NO) is a principal messenger molecule *(Moncada S. et al.: Nitric oxide: physiology, pathophysiology and pharmacology, Pharmacol. Rev. 1991. 43:109-142; Nathan C.: Nitric oxide as a secretory product of mammalian cells, FASEB J 1992. 6:3051-3063)* through which endothelium-regulated vasodilation occurs. The endothelium produces NO from L-arginine *via* an enzymatic reaction, by the endothelial NOS (Nitric Oxide Synthase). NO that is produced in the endothelium diffuses into the vascular smooth muscle cells, where it activates the production of cGMP (cyclic GMP), which in turn relaxes the constricted smooth muscle, thereby leading to vasodilation.

In addition, it has also been shown (*Mészáros L.G. et al.: Inhibition of the skeletal muscle ryanodine receptor by nitric oxide, FEBS Lett. 1996. 380:49-52; Inactivation of the cardiac ryanodine receptor calcium release channel by nitric oxide. Cell Calc. 1997. 22:447-453)* that, by decreasing Ca²⁺ "leak" from the sarcoplasmic reticulum, NO can improve the "tightness" of excitation-contraction coupling in skeletal and cardiac muscles by eliminating "unwanted" and irregular contractions, which ― under certain physiological conditions ― could also result in muscle relaxation.

We have previously reported (*Mészáros L.G. et al.: Nitric oxide suppresses spontaneous calcium release events in isolated rat ventricular myocytes. Biophys. J 1998. 74:A247*) that the availability of L-arginine for the NOS reaction might limit the proper functioning of the NO-dependent regulatory systems. We have, for instance, observed that 80-100 □M L-arginine circulating in the blood and even lower concentrations of free cellular L-arginine are suboptimal, as evidenced by the fact that further increase of L-arginine concentration in bathing solutions significantly decreases irregularity of cardiac muscle cell contractions.

In the view of the central role of L-arginine in vasodilation, there have been several uses of L-arginine suggested for both oral and topical skin applications. For example, oral administration of L-arginine have been indicated in lowering blood pressure (see: *US Patent Specification 6,458,841; 6,127,421; 5,364,884; 5,217,997*), in improving sexual functioning in both males and females (see: *US Patent Application 2003*/*0028169, 2003*/*0018076, 2002*/*0155136, 2002*/*0071854, 2002*/*0037862*) and in type II diabetes *(US Patent Specification 6,143,786*).

In addition, the use of L-arginine was also claimed to treat pulmonary edema *(US Patent Specification 5,019,558)* and to improve memory *(US Patent Secification 5,019,558*). Its dermatological use has been suggested for promoting wound healing, erectile functions, hair growth, eliminating pain and reducing weight (see: *US Patent Specification 6,458,841; 6,019,976; 6,716,436; 6,458,841; 5,883,130; 6,458,841 and US Patent Application 2003*/*0091601, 2003*/*0028169, 2002*/*0041903).* Other skin applications of L-arginine has also been claimed, which, however, utilize L-arginine as a positively charged ion or a basic amino acid that alters the physical behavior of particular cosmetic formulas.

Since the discovery of botulinum toxin as a cosmetic agent to eliminate facial wrinkles (*Mendez-Eastman SK: BOTOX: a review, Plast. Surg. Nurs. 2000, 20:60-*5), it is known that repeated movements of facial muscles over the years contribute to the development of certain types of wrinkles. The fact that botulinum toxin blocks muscle functioning, i.e. it makes muscle unable to contract, suggests that other more gentle ways of relaxing muscle, like the usage of L-arginine, for instance (see above), might indeed have similar results as far as eliminating wrinkles are concerned.

### 3. Descripton of the Invention

We have found that L-arginine, despite of its chemical character (e. g. being charged), is capable of rapidly penetrating the skin and reaching circulation, as evidenced by animal experiments and a limited number of human trials. This is the basis of our invention.

Therefore, the present invention relates to topical pharmaceutical compositions such as a cream or an ointment for the improvement of the conditions of venous insufficiencies or skeletal muscles comprising as an active agent L-arginine or any pharmacologically acceptable L-arginine derivate together with conventional excipients and/or caffeine and/or vasoactive herbal ingredients.

According to a preferred embodiment, the L-arginine concentration is in the range of 0.5 to 15% by weight and any pharmacologically acceptable L-arginine derivate is an L-arginine salt, L-arginine ester, L-arginine amid or a mixture thereof.

According to another preferred embodiment, vasoactive herbal ingredients are procyanidins, anthocyanidines, *Horse Chestnut* extract and *trans* resveratrol.

If desired, in our composition may have caffeine, its concentration ranges from about 0.1 w/w% to about 2 w/w%.

More particularly, our invention also relates to a topical use of a composition containing the above-mentioned active ingredients on the skin to improve conditions in venous insufficiencies, such as heavy and sore legs, pain, aching, inflammation, swelling, edema and the condition of the skin over varicose veins including venous ulcers and dermatitis.

A further preferred embodiment of invention is the topical use of the composition on the skin to improve conditions of the skeletal muscle, as in the case of muscle fatigue, cramps, spasms and pain, or to treat leg cramps either idiopathic, or related to either pregnancy, renal dialysis, or peripheral vascular disease (both venous and arterial), or to revitalize muscle, or to improve muscle strength after vigorous exercise and intense sport. Our composition can be used as sport creams and/or massage creams.

In another further preferred embodiment of invention, the composition is topically used on the skin in cosmetics to improve the conditions and the appearance of the skin (decreasing wrinkle depth, softening the dermal tissue and eliminating pimples and acne).

Last but not least, our composition can be used on the skin in cosmetics to improve the conditions and the appearance of the skin over areas on the thighs and buttocks of women, where the condition of the so-called cellulite exists.

### 4. Detailed information, examples:

According to our experiments, radioactive (C¹⁴-labeled) L-arginine (*Amersham Biosciences)* in concentrations of 100 µM mixed into a cream base* was topically applied onto the abdominal area of rats. Then, blood was drawn from the tail vein and analyzed for radioactivity by liquid scintillation. The results are shown in Figure 1.
*cream base: pharmacy grade liquid paraffm, 10%; crystalline paraffin, 6% g; cetylstrearate, 8% g; glycerin, 5%; sodium laurylsulfate, 1%; and distilled water, 70%)

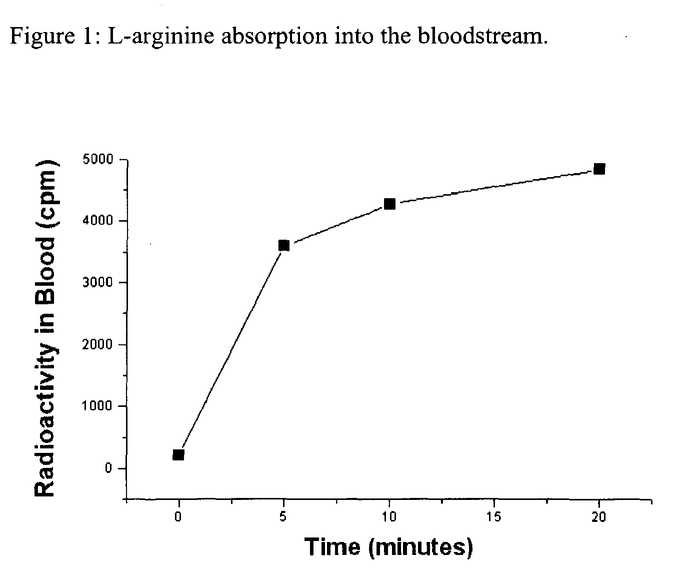

### Example

L-arginine mixed into a cream base (components: 10 w% pharmacy grade liquid paraffin, 6 w% crystalline paraffin, 8 w% cetylstrearate, 5 w% glycerin, 1 w% sodium laurylsulfate and 70w% distilled water) in concentrations 5 w% was given to volunteers to test efficacy in *(i)* eliminating pain and spasms, (*ii*) improving conditions of varicose veins and *(iii)* decreasing the depth of wrinkles on facial skin of females.

### Trial 1

22 females and 9 males received a composition containing L-arginine (see: Example) for treating various conditions of varicose veins for a period of, at least, 2 months. Based on the reports from the cream recipients, it was concluded that their general conditions had improved. They reported efficacies in:
- eliminating or decreasing swelling on the leg,
- eliminating cramps**,
- eliminating soreness of the leg and "heavy leg" feeling,
- fading of tortuosity of superficial veins in the lower extremities and
- improvement of dermatitis over visible, swollen veins.
**Many recipients of the cream reported prompt action, as experienced with the elimination of cramps.

### Trial 2

14 female handball players used a composition containing L-arginine (see: Example) for a period of 4 weeks. Based on their reports it was concluded that the cream
- decreased soreness,
- significantly reduced the feeling of fatigue of leg muscles (when applied before training or games),
- efficiently and rapidly eliminated cramps (when applied during training or games)
- accelerated healing of muscle injuries
- 12 out of the 14 recipients preferred the use of the L-arginine-containing cream over another (commercially available) sport-creams.

### Trial 3

9 women with varicose vein conditions received a composition containing L-arginine (see: Example), which also contained 1% grape seed extract (95% polyphenol content, produced by Guilin Herbal Ingredient, China). The name of this composition was: *Cream A.*

The same 9 women with varicose vein conditions received a commercially available varicose vain cream with *Horse Chestnut* extract (named: *Cream B).* The recipients were not informed about the ingredients of the creams but they were instructed to use each cream for a 1-month period in an alternating fashion with, at least, 2-week gap between the uses of the two creams. The results of the survey are shown in Table 1.

**Table 1 Comparison of the L-arginine-containing cream and a commercially available venous cream.**

| | *Cream A* | *Cream B* |
|---|---|---|
| Total number of uses | 41 | 38 |
| Improvement of all symptoms related to varicose vein condition (efficacy graded between 1 and 10)¹ | 8.7 | 5.4 |
| How fast improvement appears (1-10)^{1,2} | 9.1 | 2.3 |

| | | |
|---|---|---|
| ¹*Averaged value of the grading from 9 recipients;* ^{*2*}*Grade 10 being "fastest"*. | | |

### Trial 4

27 woman used a composition containing L-arginine (see: Example) as a facial cream for, at least, a 1-month period. After the treatment the recipients were asked to evaluate the results. In summary, they concluded that the L-arginine-containing cream
- decreased the depth of the wrinkles especially around the eyes, the mouth and on the forehead,
- increased the softness of the skin on the entire face and
- accelerated the disappearance of pimples.

## Claims

1. A topical pharmaceutical composition such as a cream or an ointment for the improvement of the conditions of venous insufficiencies or skeletal muscles comprising as the active agent L-arginine or any pharmacologically acceptable L-arginine derivate together with conventional excipients and/or caffeine and/or vasoactive herbal ingredients.

2. A composition according to claim 1, wherein the L-arginine concentration is in the range of 0.5 to 15% by weight.

3. A composition according to claim 1, wherein any pharmacologically acceptable L-arginine derivate is an L-arginine salt, L-arginine ester, L-arginine amid or a mixture thereof.

4. A composition according to claim 1, wherein vasoactive herbal ingredients are procyanidins, and/or anthocyanidines, and/or *Horse Chestnut* extract and/or *trans* resveratrol.

5. A composition according to claim 1, wherein caffeine is present from about 0.1 w/w% to about 2 w/w%.

6. A topical use of a composition according to claim 1 on the skin to improve conditions in venous insufficiencies, such as heavy and sore legs, pain, aching, inflammation, swelling, edema and the condition of the skin over varicose veins including venous ulcers and dermatitis.

7. A topical use of a composition according to claim 1 on the skin to improve conditions of the skeletal muscle, such as muscle fatigue, cramps, spasms and pain.

8. A topically use of a composition according to claim 1 on the skin to treat leg cramps either idiopathic, or related to either pregnancy, renal dialysis, or peripheral vascular disease (both venous and arterial).

9. A topical use of a composition according to claim 1 on the skin to revitalize muscle or to improve muscle strength after vigorous exercise and intense sport.

10. A topical use of a composition according to claim 1 on the skin for sport creams and/or massage creams.

11. A topical use of a composition according to claim 1 in cosmetics to improve the conditions and the appearance of the skin (decreasing wrinkle depth, softening the dermal tissue and eliminating pimples and acne).

12. A topical use of a composition according to claim 1 on the skin in cosmetics to improve the conditions and the appearance of the skin (the conditions of the so-called cellulite on the thighs and buttocks of women).
